# EUROPEAN PATENT APPLICATION

(11) **EP 1 369 809 A2**
(43) Date of publication of application: **10.12.2003**
(21) Application number: 03012616.3
(22) Date of filing: 03.06.2003
(51) Int. Cl.: G06F 19/00

(54) **Method of and system for evaluating joint and computer program for carrying out the method**

(30) Priority: 05.06.2002 JP 2002164691; 24.03.2003 JP 2003080638
(71) Applicant: Fuji Photo Film Co., Ltd., Kanagawa (JP)
(72) Inventor: Shimura, Kazuo, Ashigarakami-gun, Kanagawa-ken (JP); Osaka, Ikue, Ashigarakami-gun, Kanagawa-ken (JP)
(74) Representative: Klunker . Schmitt-Nilson . Hirsch

(57) **Abstract**

Bone image data representing a bone image including a desired joint is obtained. A region of interest including the desired joint is set on the image represented by the obtained bone image data, and a joint index representing the degree of disease of the joint in the region of interest is calculated on the basis of the part of the bone image data corresponding to the region of interest.

## Description

### BACKGROUND OF THE INVENTION

### Field of the Invention

This invention relates to a method of and a system for evaluating a joint and a computer program for carrying out the method, and more particularly to a method of and system for calculating a joint index, a system for evaluating a joint, and a method of and system for outputting change with time of a joint which are suitable for diagnosing inflammation or rheumatism of a finger joint, and a computer program for the systems and methods.

### Description of the Related Art

There has been used a radiation image in diagnosis of inflammation or rheumatism of a joint. Especially in diagnosis of rheumatism, the doctor judges progress of rheumatism by scoring the state of bone fracture over a radiation image of the joint. As a technology of scoring the state of bone fracture, there has been known, for instance, a technology disclosed in "Arvin Larsen: How to Apply Larsen Score in Evaluating Radiographs of Rheumatoid Arthritis in Long Term Studies, The Journal of Rheumatology 1995".

However there has been a problem that the conventional technology where progress of rheumatism is judged by visually scoring the state of bone fracture over a radiation image of the joint is poor in objectivity and reproducibility and according to the conventional technology, evaluation of the joint varies from doctor to doctor.

### SUMMARY OF THE INVENTION

In view of the foregoing observations and description, the primary object of the present invention is to provide a method of and system for calculating a joint index, a system for evaluating a joint, and a method of and system for outputting change with time of a joint which are higher in objectivity and reproducibility, and a computer program for the systems and methods.

In accordance with a first aspect of the present invention, there is provided a method of calculating a joint index comprising the steps of
obtaining bone image data representing a bone image including a desired joint,
setting a region of interest including the desired joint on the image represented by the obtained bone image data, and
calculating a joint index representing the degree of disease of the joint in the region of interest on the basis of the part of the bone image data corresponding to the region of interest.

In accordance with a second aspect of the present invention, there is provided a system for calculating a joint index comprising
an image obtaining means which obtains bone image data representing a bone image including a desired joint,
an interesting region setting means which sets a region of interest including the desired joint on the image represented by the obtained bone image data, and
an index calculating means which calculates a joint index representing the degree of disease of the joint in the region of interest on the basis of the part of the bone image data corresponding to the region of interest.

"The joint index" as used here means a physical value which represents the state of a given joint calculated on the basis of the part of the bone image data corresponding to the region of interest according to a predetermined method of calculation and on the basis of which the degree of disease of the joint is calculated. Such a joint index may be, for instance, a value representing the thickness of bones forming the joint, a value representing the recess in the bones (the area over which the bone is dissolved or the number of dissolved parts) or a value representing the gap between opposed edges of the bones forming the joint.

When the bone image data obtained by the image obtaining means represents an image of a hand bone including a finger joint, the interesting region setting means may comprise, for instance, a hand region extracting means which extracts a part of image data representing the hand region from the image data obtained by the image data obtaining means on the basis of the values of density signals of the image data, a morphology processing means which repeats morphology processing on the extracted part of image data representing the hand region until the finger region disappears, and a finger region extracting means which extracts a part of image data representing the finger region as the region of interest on the basis of the difference between the finger-region-free hand region image (the image of the hand region from which the finger region disappears) enlarged to the original size and the hand region image before morphology processing.

The index calculating means may calculates a plurality of different indexes each representing the degree of disease of the joint, and may calculate a new index on the basis of the plurality of indexes.

The system in accordance with the second aspect of the present invention may further comprise a storage means which stores the index relating to information which identifies the joint from which the index is calculated. For example, information for identifying the patient such as an ID number is attached to the bone image data, a different label is attached to each set region of interest, and the bone image data is stored together with the label and the calculated index attached to the bone image data.

The system in accordance with the second aspect of the present invention may further comprise a display means which displays an image on its screen. The display means displays, for instance, the region of interest set by the interesting region setting means together with the bone image, the value of the index calculated by the index calculating means near the corresponding joint in the bone image or in a region separate from the region where the bone image is displayed, or a comment according to the value of the index.

The system in accordance with the second aspect of the present invention may further comprise a hard copy output means which outputs an image as a hard copy recorded on a recording medium such as paper or film. The hard copy output means may output the image displayed by the display means, the images and the indexes one by one, or the image and the index together.

In accordance with a third aspect of the present invention, there is provided a system for evaluating a joint comprising
an image obtaining means which obtains bone image data representing a bone image including a desired joint,
an interesting region setting means which sets a region of interest including the desired joint on the image represented by the obtained bone image data,
an index calculating means which calculates at least one joint index representing the degree of disease of the joint in the region of interest on the basis of the part of the bone image data corresponding to the region of interest, and
an evaluation means which evaluates the degree of disease of the joint in the region of interest on the basis of the index calculated by the index calculating means.

The "evaluation" as used here means evaluation in a clinical sense. For example, progress of rheumatism may be evaluated by the level.

In accordance with a fourth aspect of the present invention, there is provided a system for evaluating a joint comprising
an image obtaining means which obtains bone image data representing a bone image including a plurality of joints in a predetermined part,
an interesting region setting means which sets a plurality of regions of interest each including a desired joint on the image represented by the obtained bone image data,
an index calculating means which calculates at least one joint index representing the degree of disease of the joint in each region of interest on the basis of the part of the bone image data corresponding to the region of interest, and
an evaluation means which evaluates the degree of disease of the predetermined part on the basis of the indexes calculated for the respective joints by the index calculating means.

The "predetermined part" may be, for instance, the left or right hand of the patient or the left or right foot of the patient and "to evaluate the degree of disease of the predetermined part" means, for instance, "to evaluate the degree of progress of rheumatism of the left hand of the patient by the level on the basis of the indexes for the finger joints of the left hand of the patient".

The evaluation means either may evaluate the degree of disease of the predetermined part directly on the basis of the indexes calculated for the respective joints or may once evaluate the degree of disease of the respective joints on the basis of the indexes calculated for the respective joints and evaluate the degree of disease of the predetermined part on the basis of the result of evaluation on the respective joints.

In accordance with a fifth aspect of the present invention, there is provided a system for evaluating a joint comprising
an image obtaining means which obtains a plurality of pieces of bone image data representing a plurality of images of bones including a joint in different parts of a patient,
an interesting region setting means which sets at least one region of interest including a desired joint on each of the images represented by the plurality of pieces of bone image data,
an index calculating means which calculates at least one joint index representing the degree of disease of the joint in each region of interest on the basis of the part of the bone image data corresponding to the region of interest, and
an evaluation means which evaluates the degree of disease of the patient on the basis of the indexes calculated for the respective joints by the index calculating means.

The "different parts of a patient" may be one and the other of parts, e.g., the left and right hands, or the left and right feet, as well as elbow and knee or hands and feet. To "evaluate the degree of disease of the patient" means, for instance, "to evaluate the degree of entire progress of rheumatism of the patient by the level on the basis of the indexes for the finger joints of the left and right hands and left and right feet of the patient".

In a method of outputting change with time of a joint in accordance with a sixth aspect of the present invention,
a plurality of joint indexes are calculated on a bone joint by a method of calculating a joint index of the first aspect of the present invention on the basis of a plurality of pieces of image data representing the image of the bone joint taken at different times, the indexes are stored related to information which identifies the joint from which the indexes are calculated, the joint indexes are searched for on the basis of the information and the change with time of the index is output on the basis of the plurality of indexes obtained.

A system for outputting change with time of a joint in accordance with a seventh aspect of the present invention comprises
a storage means which stores a plurality of joint indexes calculated on a bone joint by a method of calculating a joint index of the first aspect of the present invention on the basis of a plurality of pieces of image data representing the image of the bone joint taken at different times relating to information which identifies the joint from which the indexes are calculated,
an index obtaining means which searches for the joint indexes on the basis of the information, and
an output means which outputs the change with time of the index on the basis of the plurality of indexes obtained.

The output means may output the change with time of the index in various forms. For example, the output means may output the joint indexes obtained from images taken at different times, as they are, the difference between two of the indexes or the degree of improvement or deterioration in disease of the bone joint calculated on the basis of the difference between two of the indexes.

The output means may comprise a display means which displays an image on its screen and a hard copy output means which outputs an image as a hard copy recorded on a recording medium such as paper or film. The display means may display, for instance, the images of the bones taken at different times together with the indexes calculated on the basis of the images, the change with time of the index, or a comment according to the value of the index. The hard copy output means may output the image displayed by the display means, the images and the indexes one by one, or the image and the index together.

A computer program for causing a computer to perform the method of the present invention may be installed in a computer. Otherwise such a computer program may be recorded in a computer readable medium so that the computer can perform the method when loaded with the recording medium. A skilled artisan would know that the computer readable medium is not limited to any specific type of storage devices and includes any kind of device, including but not limited to CDs, floppy disks, RAMs, ROMs, hard disks, magnetic tapes and internet downloads, in which computer instructions can be stored and/or transmitted. Transmission of the computer code through a network or through wireless transmission means is also within the scope of this invention. Additionally, computer code/instructions include, but are not limited to, source, object and executable code and can be in any language including higher level languages, assembly language and machine language.

The "bone joint" as used here need not be limited to those of a human body but may be those of an animal body.

The " image" as used here should be broadly interpreted to include image data as well as a visible image actually displayed. For example, an expression "to obtain an image" includes to merely obtain image data on the basis of which an image is displayed.

In accordance with the method of and the system for calculating a joint index of the first and second aspects of the present invention, an index representing the degree of disease of the bone joint is calculated according to a predetermined calculation on the basis of image data representing an image of the bone joint and the state of the joint can be numerically expressed. Accordingly, by the use of the calculated joint index, evaluation higher in objectivity and reproducibility as compared with the conventional technology where the degree of disease is visually evaluated can be realized.

When the system for calculating a joint index of the second aspect of the present invention further comprises a storage means which stores the index relating to information which identifies the joint from which the index is calculated, the index or indexes calculated in the past can be obtained on the basis of the information which identifies the joint from which the index is calculated, and accordingly, it is not necessary to calculate again an index which has been once calculated.

In accordance with the system for evaluating a joint of the third aspect of the present invention, an index representing the degree of disease of the bone joint is calculated according to a predetermined calculation on the basis of image data representing an image of the bone joint and the degree of disease of the joint is evaluated on the basis of the calculated index. Accordingly, evaluation of the bone joint can be effected in a manner which substantially corresponds to clinical evaluation generally carried out by readers and is more understandable, as well as higher in objectivity and reproducibility.

In accordance with the system for evaluating a joint of the fourth aspect of the present invention, joint indexes representing the degrees of disease of the joints in a predetermined part are calculated, and the degree of disease of the predetermined part is evaluated on the basis of the indexes calculated for the respective joints. Accordingly, not only each of bone joints but also the part formed by the bone joints can be evaluated and the demand of readers, e.g., doctors, for information on generic evaluation of joints by part can be met.

In accordance with the system for evaluating a joint of the fifth aspect of the present invention, joint indexes representing the degrees of disease of the joints in parts of a patient are calculated, and the degree of disease of the patient is evaluated on the basis of the indexes calculated for the respective joints. Accordingly, not only each of bone joints of the patient but also the patient having the bone joints can be evaluated and the demand of readers, e.g., doctors, for information on generic evaluation of joints by patient can be met.

In accordance with the method of and the system for outputting change with time of a joint of the sixth and seventh aspects of the present invention, the joint indexes of different times which have been calculated and stored are searched for on the basis of the information which identifies the joint from which the index is calculated and the change with time of the index is output on the basis of the plurality of indexes obtained. Accordingly, the degree of improvement or deterioration in disease of the bone joint can be grasped and the degree of wider disease of joint can be evaluated.

### BRIEF DESCRIPTION OF THE DRAWINGS

Figure 1 is a view showing in brief a bone evaluation system in accordance with an embodiment of the present invention,
Figures 2A to 2E and Figure 3 are views for illustrating the procedure of extracting the finger image data from the radiation image data P,
Figures 4A and 4B are views for illustrating a way of setting and labeling the regions of interest,
Figure 5 shows the object image on which the regions of interest have been set and labeled,
Figures 6A to 6D are views for illustrating a method of counting the number of the recessed portions and measuring the depths of the recessed portions,
Figure 7 is a view showing the gap between opposed edges of the bones forming the joint,
Figures 8A to 8F are views respectively showing the grades of the degree of progress of rheumatism,
Figure 9 is a view showing an example of the display by the display means,
Figure 10 is a view showing another example of the display by the display means, and
Figure 11 is a view showing still another example of the display by the display means.

### DESCRIPTION OF THE PREFERRED EMBODIMENT

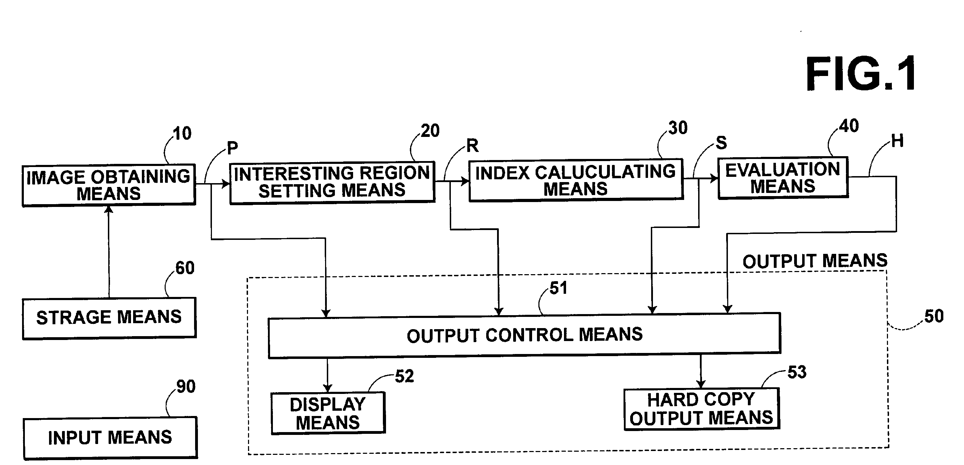

In Figure 1, a bone evaluation system in accordance with an embodiment of the present invention comprises an image data obtaining means 10 which obtains image data P representing a bone image including a joint, an interesting region setting means 20 which sets a region of interest R including the joint on the image represented by the obtained bone image data P, an index calculating means 30 which calculates at least one index S representing the degree of disease of the joint in the region of interest R on the basis of the part of the bone image data P corresponding to the region of interest R, an evaluation means 40 which evaluates the degree of disease of the joint on the basis of the calculated index, an output means 50 which outputs a medical image represented by the obtained image data P, the set region of interest R, the calculated index S and the evaluated degree H of disease of the joint, a storage means 60 which stores the obtained image data P together with information on the patient having the joint represented by the image data P (e.g., an ID number), information on the image (e.g., the object part, the date of taking the image P and the file number) and information on the joint (e.g., the size and/or the position of the set region of interest, the calculated index and the evaluated degree of disease of the joint) and an input means 90 for controlling the system.

The out means 50 further comprises an output control means 51 which controls output of the medical image represented by the bone image data P, the set region of interest R, the calculated index S and the evaluated degree of disease H, a display means 52 such as a CRT or a liquid crystal display which displays these pieces of output information, and a hard copy output means 53 such as a printer which outputs an image as a hard copy recorded on a recording medium such as paper or film.

The "index" as used here means a physical value which represents the state of a given joint calculated on the basis of the part of the bone image data corresponding to the region of interest according to a predetermined method of calculation and on the basis of which the degree of disease of the joint is calculated. The "evaluation" as used here means evaluation in a clinical sense.

Operation of the bone evaluation system of this embodiment of an arrangement described above will be described hereinbelow. In the following description, it is assumed that rheumatism of a finger joint is the suspected disease and a radiation image of a hand including a finger joint is input as a bone image data . For instance, an image of a hand including the finger joint obtained by CT or MRI may be used in place of the radiation image.

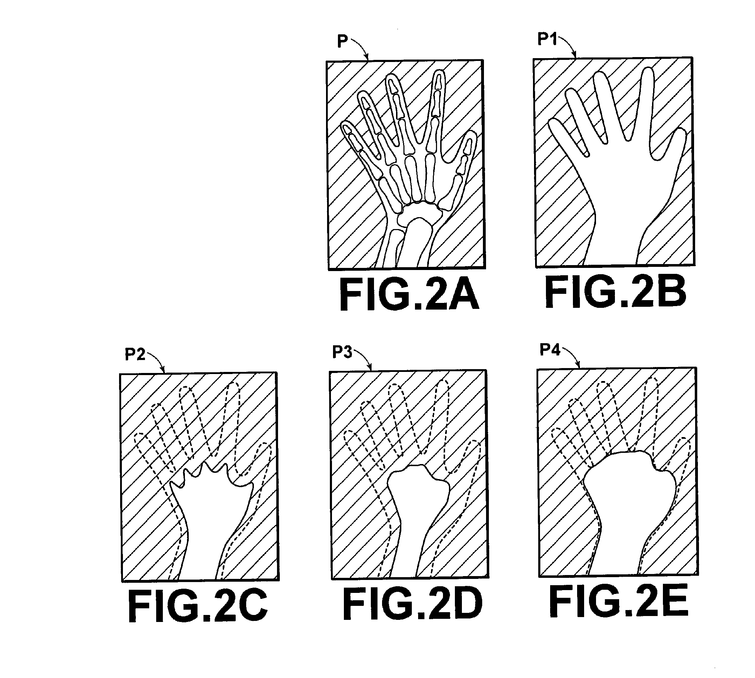

The image obtaining means 19 obtains bone image data P representing a radiation image of a left hand including a finger joint such as shown in Figure 2A from, for instance, an image read-out system (not shown) or the storage means 60. The image data P can be attached with information on the patient having the joint represented by the image data P (e.g., an ID number), information on the image (e.g., the object part, the date of taking the image P and the file number) and information on the joint(e.g., the size and/or the position of the set region of interest, the calculated index and the evaluated degree of disease of the joint) and the image obtaining means 10 may search for an image on the basis of these pieces of information.

The interesting region setting means sets a region of interest R(i,j) on the image represented by the obtained bone image data P, i standing for the kind of the finger, e.g., 1 representing a little finger, 2 representing a ring finger, 3 representing a middle finger, 4 representing an index finger and 5 representing a thumb, and j standing for the kind of the joint, e.g., 1 representing a first joint and 2 representing a second joint. The region of interest R may be manually set, for instance, by designating a rectangular frame by the input means 90 or automatically set by carrying out an image analysis on the obtained image data P. The region of interest R can be automatically set, for instance, in the following manner.

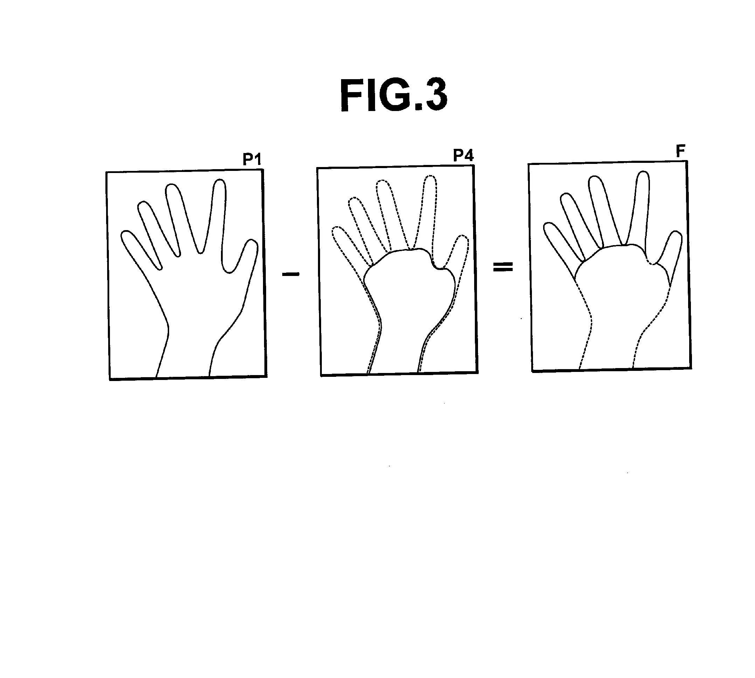

That is, finger image data representing only the fingers is extracted from the radiation image data P representing the image of hand including joints, and a region of interest is set on each of the finger joints on the radiation image on the basis of the extracted finger image data (part of the image data P representing the fingers) . Figures 2A to 2E and Figure 3 are views for illustrating the procedure of extracting the finger image data from the radiation image data P. First the radiation image data P representing an image of a hand as shown in Figure 2A is binary-coded to divide the radiation image P into a hand region and a background region, thereby obtaining a hand region image data P1 representing the hand region as shown in Figure 2B. A processing for reducing the hand region (to obtain image data P2 representing a reduced hand region as shown in Figure 2C) is carried out on the hand region image data P1 until the finger region disappears, thereby obtaining finger-less image data P3 representing an image without finger as shown in Figure 2D. Then, the finger-less image P3 is enlarged to such an extent that the back of the hand of the image represented by the image data P3 is equivalent to that represented by the image data P1 in size as shown in Figure 2E, thereby obtaining an enlarged finger-less image data P4. Then finger region image data F representing the fingers is obtained by subtracting image data P4 from the image data P1 (subtracting from a value of each pixel of the image data P1 a value of the corresponding pixel of the image data P4) as shown in Figure 3.

For example, the hand region is reduced by morphology processing. The morphology processing includes dilation for searching for a maximum pixel value in a range of a predetermined width about a pixel of interest in the image and erosion for searching for a minimum pixel value in a range of a predetermined width about a pixel of interest in the image. In a high-brightness, high-signal-level image, edges of regions having a high brightness is deleted and the image is reduced when the erosion is carried out on the image data. In a high-brightness, low-signal-level image, edges of regions having a high brightness is deleted and the image is reduced when the dilation is carried out on the image data. In this particular embodiment, the hand region image data P1 is assumed to be expressed by a high-brightness, high-signal-level image signal. In this case, by repeating the erosion on the hand region image data, the edges of the hand region is eroded and the profile of the hand region is reduced. The morphology processing is discussed in detail, for instance, in Japanese Unexamined Patent Publication No. 2002-109510.

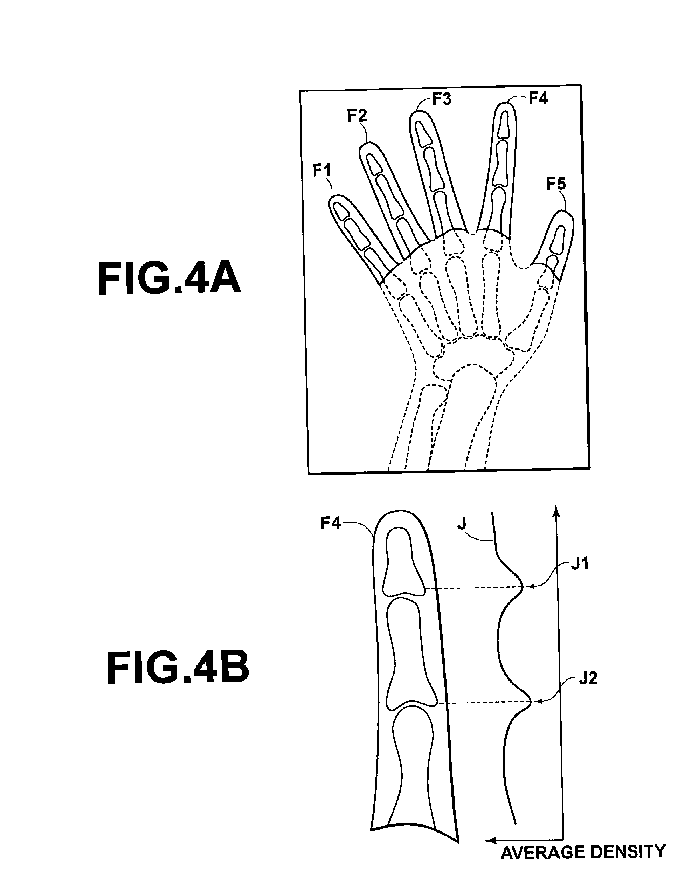

Then the extracted finger region image data F representing the fingers is further divided into parts representing each of the fingers, the thumb, the index finger, the middle finger, the ring finger and the little finger on the basis of the image information, e.g., relative positions, the size and/or the length, and the images of the respective fingers are labeled as shown in Figure 4A, where labels F1 to F5 respectively indicate the thumb to the little finger. Then densities of pixels arranged in perpendicular to the length of the finger are averaged, and the averages are plotted in the direction of the length of the finger, thereby obtaining an average density curve. In a radiation image, since bones generally absorb radiation, pixels in the bone region is low in density, and as the finger becomes thicker, the average density becomes smaller. Accordingly, in a region of a joint where the bone becomes locally thick, the average density locally becomes small. Accordingly, by detecting a peak of the average density curve, the position of a joint can be identified. Then regions of interest can be set by setting predetermined sizes of frames about the positions of the joints . Figure 4B shows the image F4 of the index finger and the average density curve J of the image F4 . In Figure 4, J1 and J2 indicate peaks of the average density curve J.

Otherwise, the region of interest R can be automatically set in the following manner. In this method, past image data representing an image of the same object joint which has been taken in the past (will be referred to as "a past image", hereinbelow) and information attached to the past image data. When the regions of interest have been once set on an image and there is image data attached with information representing the positions and the sizes of the regions of interest, the past image data is obtained by searching, for instance, the storage means 60, and the images represented by the object image data and the past image data are superposed one on the other. Coordinates on the object image corresponding to the regions of interest on the past image are obtained, and regions of interest can be set on the object image on the basis of the coordinates. When regions of interest are once set on an image, it is not necessary to set again regions of interest on images which are subsequently taken. This procedure is better in reproducibility of the positions of the regions of interest. When there is no past image, regions of interest can be set by superposing the object image on an image having a standard shape common to the object so long as the shape of the object (hand in this particular embodiment) is substantially constant.

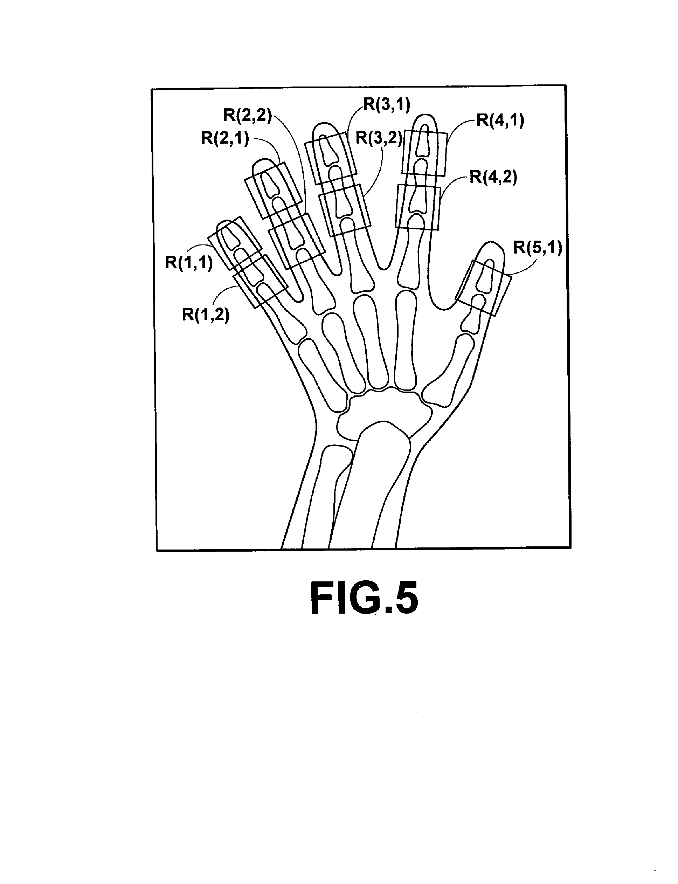

Figure 5 shows the object image P on which the regions of interest R have been set and labeled. The index calculating means 30 calculates indexes S(i, j, k, m) representing the degree of progress of rheumatism in the regions of interest R(i, j) on the basis of the part of the bone image data corresponding to the regions of interest, k representing a number corresponding to the kind of the index, and m representing a number for distinguishing the part for which the index is to be calculated in the case where there are plurality of parts for which the index is to be calculated. In this particular embodiment, the average density (the average radiation absorption) S(i, j, 1) of the bone region representing the thickness of the bone; the number of the recessed portions S(i, j, 2) and the size of each recessed portion S(i, j, 3, m) which represent the degree of generation of recessed portion in the bone; and the gap between opposed edges of the bones forming the joint S(i, j, 4) are calculated as the indexes.

The index S(i, j, 1) representing the thickness of the bone is the average density of pixels arranged in perpendicular to the length of the finger across the joint described above in conjunction with detection of the joint. Since the bone region absorbs more radiation and is expressed as a low density portion in a radiation image, the average density of pixels arranged in perpendicular to the length of the finger across the joint may be regarded as an index quantifying the thickness of the joint relatively to the thickness of the finger.

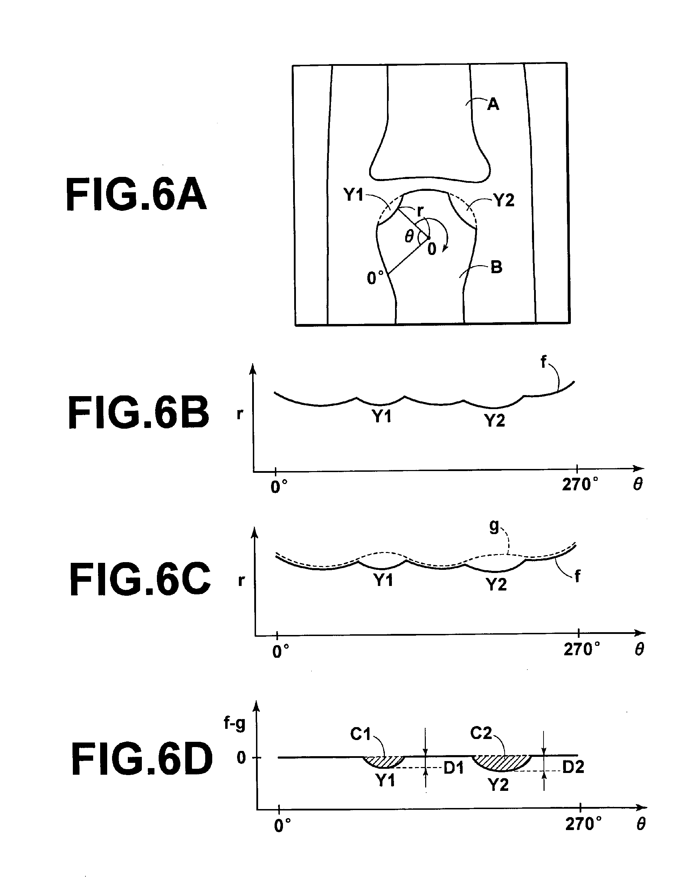

The number of the recessed portions S(i, j, 2) and the size of each recessed portion S(i, j, 3, m) are valued by measuring the number of the recessed portions and the size (depth) of each recessed portion on the basis of difference information representing the difference from an assumed normal profile of the joint extracted from the image of the joint on the head of the bone forming the joint. Figure 6A shows a joint formed by a bone A and a bone B having recessed portions Y1 and Y2. The profile of the bone B is first approximated by a circle or an ellipse and determines a center O. Then the profile of the bone B is expressed on a polar co-ordinate system having its axis on the center O, thereby obtaining a curve f shown in Figure 6B. In Figure 6A, θ represents an angle and r represents a radius. Then multidimensional polynomial approximation is carried out on the curve f to obtain a curve g shown in Figure 6C and the curve g is considered to be a normal profile. Then a difference curve f-g representing the difference between the curves f and g. As shown in Figure 6D, the recessed portions Y1 and Y2 are represented by parts of the curve f-g deviated from the straight line f-g=0. The number of recessed portions S(i, j, 2) is determined on the basis of the areas C1 and C2 of the regions circumscribed by the straight line f-g=0 and the curve f-g and the depths D1 and D2 of the regions are taken as the depths of the recessed portions S(i, j, 3, 1) and S(i, j, 3, 2).

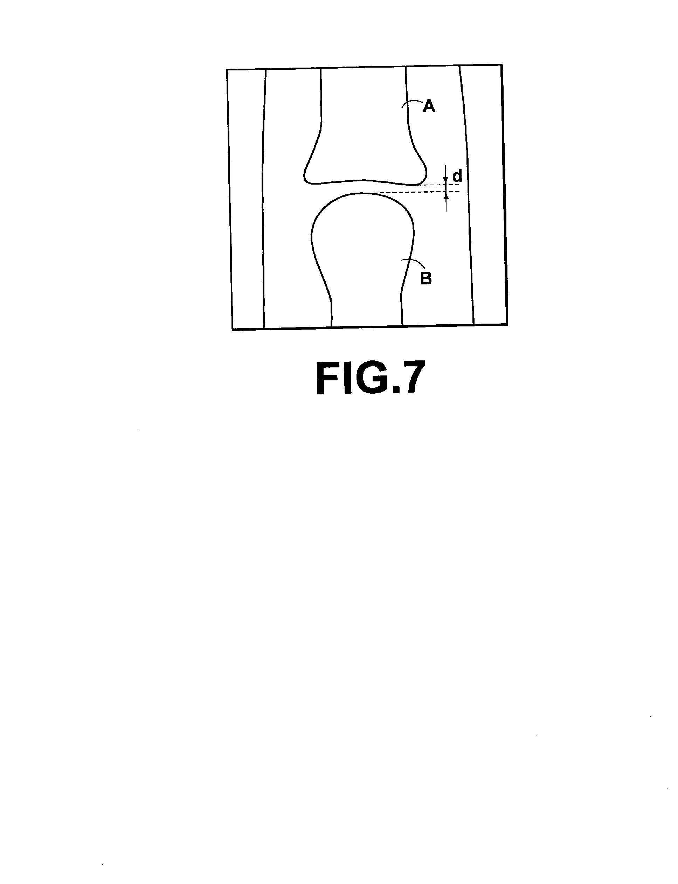

As shown in Figure 7, the gap between opposed edges of the bones forming the joint S(i, j, 4) represents the gap d between the opposed edges of the bone A and the bone B.

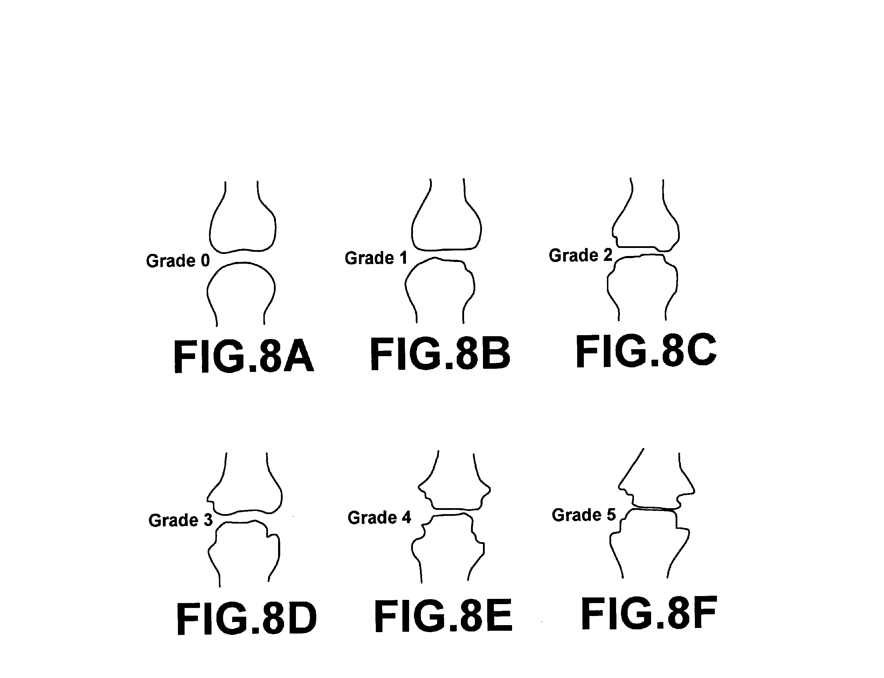

The evaluation means 40 evaluates the degree of progress of rheumatism of the joint on the basis of the calculated indexes by the clinical grades . The following grades have been employed wide. Figures 8A to 8F respectively show the following 6 grades.
Grade 0: the profile of the joint entirely remains
Grade 1: the gap between the bones is small and a dissolution not larger than 1mm exists
Grade 2: at least one dissolution larger than 1mm exists
Grade 3: a large dissolution exists
Grade 4: several dissolutions exist and the profile of the joint partly remains
Grade 5: the profile of the joint is entirely lost and the joint has been deformed

However, since the above evaluation standard includes abstract expressions, it is necessary to arrange the evaluation standard so that the degree of progress of rheumatism of the joint can be quantitatively determined on the basis of the calculated indexes. In this particular embodiment, points are added up according to the values of the index on the basis of the standard shown in the following table 1, and the degree of progress of rheumatism of the joint is judged in the following manner on the basis of the total point.

| point | index | | |
|---|---|---|---|
| | number of recesses S(i, j, 2) | depth of recess S(i, j, 3, m) | gap S(i, j, 4) |
| 0 | S ( i , j , , 2 ) = 0 | S(i, j, 3, m)max<TH31 | TH42≦S(i, j, 4) |
| 1 | 1≦ S ( i , j , 2) ≦ 2 | TH31≦S(i, j, 3, m)max<TH32 | TH41≦S(i, j, 4)<TH42 |
| 2 | 3 ≦ S (i, j , 2 ) | TH32≦S(i, j, 3, m)max | S(i, j, 4)<TH41 |

Total point: Evaluation
0:Grade 0
1 ∼ 2: Grade 1
3: Grade 2
4: Grade 3
5: Grade 4
6: Grade 5

TH31 represents a value corresponding to 1mm, TH 32 represents a predetermined value over which the dissolution is determined to be large, and TH41 represents a gap smaller than which the gap is determined to be narrow. Further, S(i, j , 3, m) max represents a maximum value of the depth in the depths of a plurality of recessed portions in the joint. The index S(i, j, 1) representing the thickness of the joint is not used here.

After all the joints are evaluated, the output control means 51 causes the display means 52 to display the indexes and the evaluation for each joint together with the image thereof and causes the storage means 60 to store the same information and/or causes the hard copy output means 53 to output the same information as a hard copy under the control of instruction input through the input means 90.

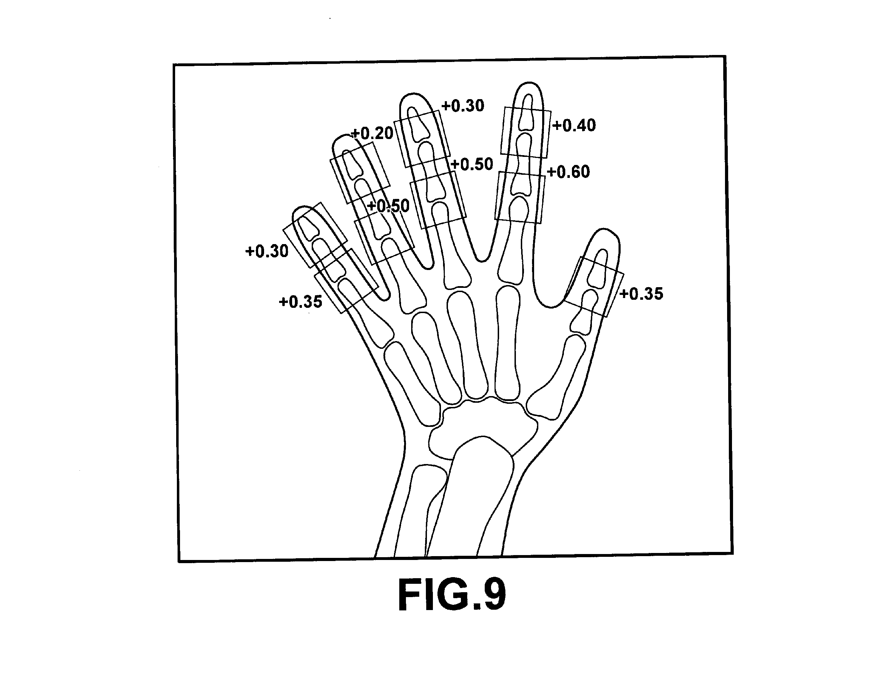

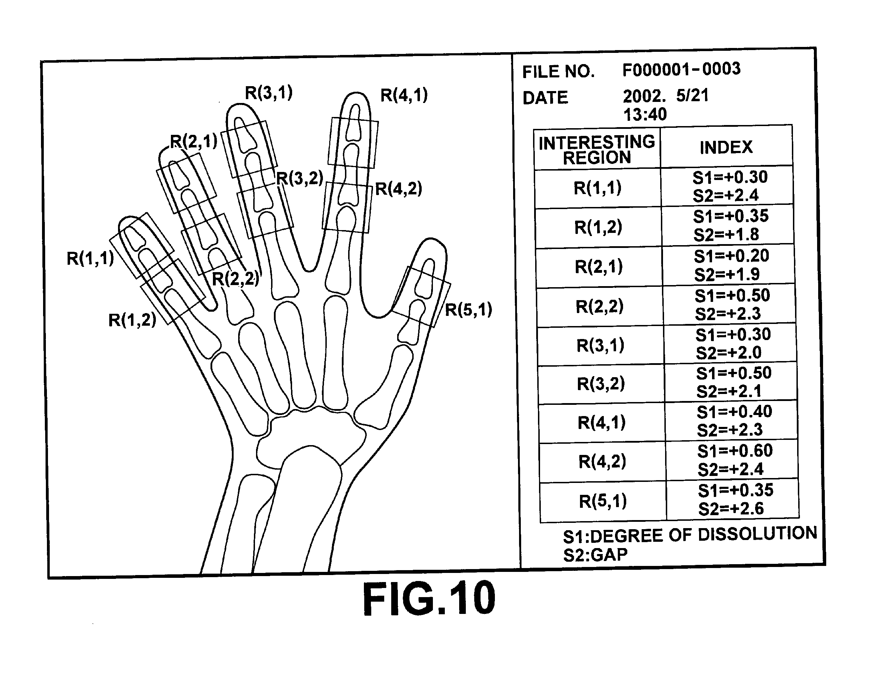

The output control means 51 can cause the display means (e.g., a CRT or a liquid crystal display) 52 to display information in various patterns. For example, the output control means 51 may cause the display means 52 to display an index near to the image of each joint on the hand region image P as shown in Figure 9, or may cause the display means 52 to display an index and/or other information in an area separate from the area in which the hand region image P is displayed as shown in Figure 10. Further, it is possible to arrange the output control means 51 to cause the display means 52 to display a region designated through the input means 90 in an enlarged scale.

Further, the output control means 51 causes the storage means 60 to store data on each joint such as the obtained image data P, information on the patient having the joint represented by the image data P (e.g., an ID number), information on the image (e.g., the object part, the date of taking the image P and/or the file number) and information on the joint (e.g., the size and/or the position of the set region of interest, the calculated index and/or the evaluated degree of disease of the joint) .

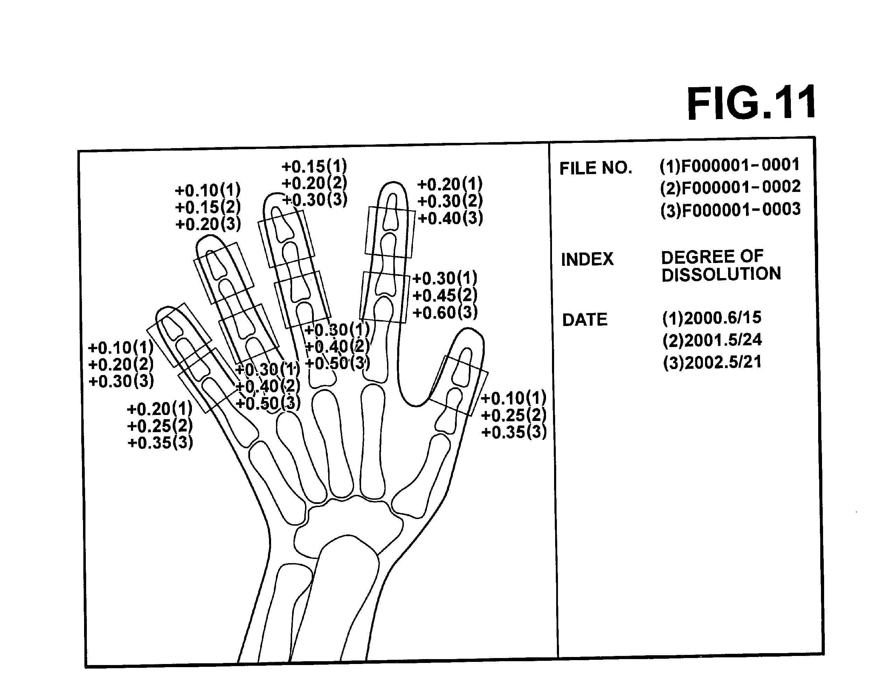

In diagnosis of rheumatism or the like of a joint, it is sometimes necessary to know progress or improvement of the disease. In such a case, the image obtaining means 10 may search the storage means 60 for an image which is taken from the same object part at a different time and the information attached to the image on the basis of the information on the patient, and the output control means 51 may cause the display means 52 to display the images taken at different times or to display in a time series indexes calculated at different times for each joint or to display the difference between the images or the indexes so that the change with time of the state of the joint can be known from the image on the screen. For example, as shown in Figure 11, the display means 52 may display indexes taken at different times near to the image of each joint and information on the date of taking each index relating to the index. The date of taking the index can be related to the index, for instance, by attaching the same numeral or sign or by displaying in the same color.

The output control means 51 further causes the hard copy output means 53 to output the same information as a hard copy.

Though, in the embodiment described above, the degree of progress of rheumatism of each joint is evaluated in a plurality of grades, the degree of progress of rheumatism of a part (e.g., the left hand of a patient) may be evaluated in a plurality of grades on the basis of the indexes calculated for the joints in the part (e.g., the left hand) . In this case, the image obtaining means obtains bone image data representing a bone image including a plurality of joints in a predetermined part, the interesting region setting means sets a plurality of regions of interest each including a desired joint on the image represented by the obtained bone image data, the index calculating means calculates at least one joint index representing the degree of disease of the joint in each region of interest on the basis of the part of the bone image data corresponding to the region of interest, and the evaluation means evaluates the degree of disease of the predetermined part on the basis of the indexes calculated for the respective joints.

Further, the degree of progress of rheumatism of a patient may be evaluated in a plurality of grades on the basis of the indexes calculated for the joints in different parts of the patient (e.g., the left and right hands of the patient) . In this case, the image obtaining means obtains a plurality of pieces of bone image data representing a plurality of images of bones including a joint in different parts of a patient, the interesting region setting means sets at least one region of interest including a desired joint on each of the images represented by the plurality of pieces of bone image data, the index calculating means calculates at least one joint index representing the degree of disease of the joint in each region of interest on the basis of the part of the bone image data corresponding to the region of interest, and the evaluation means evaluates the degree of disease of the patient on the basis of the indexes calculated for the respective joints.

With these arrangements, not only the degree of progress of rheumatism of each joint but also the degree of progress of rheumatism of a part formed by the joints or of a patient can be evaluated and the demand of readers, e.g., doctors, for information on generic evaluation of the degree of progress of rheumatism of joints by part or by patient can be met.

## Claims

1. A method of calculating a joint index comprising the steps of
obtaining bone image data representing a bone image including a desired joint,
setting a region of interest including the desired joint on the image represented by the obtained bone image data, and
calculating a joint index representing the degree of disease of the joint in the region of interest on the basis of the part of the bone image data corresponding to the region of interest.

2. A method of outputting change with time of a joint comprising the steps of ,
calculating a plurality of joint indexes on a bone joint by a method of calculating a joint index as defined in Claim 1 on the basis of a plurality of pieces of image data representing the image of the bone joint taken at different times,
storing the indexes relating to information which identifies the joint from which the indexes are calculated,
searching for the joint indexes on the basis of the information, and
outputting the change with time of the index on the basis of the plurality of indexes obtained.

3. A system for calculating a joint index comprising
an image obtaining means which obtains bone image data representing a bone image including a desired joint,
an interesting region setting means which sets a region of interest including the desired joint on the image represented by the obtained bone image data, and
an index calculating means which calculates a joint index representing the degree of disease of the joint in the region of interest on the basis of the part of the bone image data corresponding to the region of interest.

4. A system as defined in Claim 3 in which joint index represents the thickness of bones forming the joint.

5. A system as defined in Claim 3 in which joint index represents the recess in the bones forming the joint.

6. A system as defined in any one of Claims 3 to 5 further comprising a storage means which stores the index relating to information which identifies the joint from which the index is calculated.

7. A system for evaluating a joint comprising
an image obtaining means which obtains bone image data representing a bone image including a desired joint,
an interesting region setting means which sets a region of interest including the desired joint on the image represented by the obtained bone image data,
an index calculating means which calculates at least one joint index representing the degree of disease of the joint in the region of interest on the basis of the part of the bone image data corresponding to the region of interest, and
an evaluation means which evaluates the degree of disease of the joint in the region of interest on the basis of the index calculated by the index calculating means.

8. A system for evaluating a joint comprising
an image obtaining means which obtains bone image data representing a bone image including a plurality of joints in a predetermined part,
an interesting region setting means which sets a plurality of regions of interest each including a desired joint on the image represented by the obtained bone image data,
an index calculating means which calculates at least one joint index representing the degree of disease of the joint in each region of interest on the basis of the part of the bone image data corresponding to the region of interest, and
an evaluation means which evaluates the degree of disease of the predetermined part on the basis of the indexes calculated for the respective joints by the index calculating means.

9. A system for evaluating a joint comprising
an image obtaining means which obtains a plurality of pieces of bone image data representing a plurality of images of bones including a joint in different parts of a patient,
an interesting region setting means which sets at least one region of interest including a desired joint on each of the images represented by the plurality of pieces of bone image data,
an index calculating means which calculates at least one joint index representing the degree of disease of the joint in each region of interest on the basis of the part of the bone image data corresponding to the region of interest, and
an evaluation means which evaluates the degree of disease of the patient on the basis of the indexes calculated for the respective joints by the index calculating means.

10. A system for outputting change with time of a joint comprising
a storage means which stores a plurality of joint indexes calculated on a bone joint by a method of calculating a joint index of the first aspect of the present invention on the basis of a plurality of pieces of image data representing the image of the bone joint taken at different times relating to information which identifies the joint from which the indexes are calculated,
an index obtaining means which searches for the joint indexes on the basis of the information, and
an output means which outputs the change with time of the index on the basis of the plurality of indexes obtained.

11. A computer program for causing a computer to perform a procedure comprising the steps of
obtaining bone image data representing a bone image including a desired joint,
setting a region of interest including the desired joint on the image represented by the obtained bone image data, and
calculating a joint index representing the degree of disease of the joint in the region of interest on the basis of the part of the bone image data corresponding to the region of interest.

12. A computer program for causing a computer to perform a procedure comprising the steps
obtaining means bone image data representing a bone image including a desired joint,
setting a region of interest including the desired joint on the image represented by the obtained bone image data,
calculating at least one joint index representing the degree of disease of the joint in the region of interest on the basis of the part of the bone image data corresponding to the region of interest, and
evaluating the degree of disease of the joint in the region of interest on the basis of the index calculated by the index calculating means.

13. A computer program for causing a computer to perform a procedure comprising the steps of
obtaining bone image data representing a bone image including a plurality of joints in a predetermined part,
setting means a plurality of regions of interest each including a desired joint on the image represented by the obtained bone image data,
calculating at least one joint index representing the degree of disease of the joint in each region of interest on the basis of the part of the bone image data corresponding to the region of interest, and
evaluating the degree of disease of the predetermined part on the basis of the indexes calculated for the respective joints by the index calculating means.

14. A computer program for causing a computer to perform a procedure comprising the steps of
obtaining means a plurality of pieces of bone image data representing a plurality of images of bones including a joint in different parts of a patient,
setting at least one region of interest including a desired joint on each of the images represented by the plurality of pieces of bone image data,
calculating at least one joint index representing the degree of disease of the joint in each region of interest on the basis of the part of the bone image data corresponding to the region of interest, and
evaluating the degree of disease of the patient on the basis of the indexes calculated for the respective joints by the index calculating means.

15. A computer program for causing a computer to perform a procedure comprising the steps of
calculating a plurality of joint indexes on a bone joint by a method of calculating a joint index as defined in Claim 1 on the basis of a plurality of pieces of image data representing the image of the bone joint taken at different times,
storing the indexes relating to information which identifies the joint from which the indexes are calculated,
searching for the joint indexes on the basis of the information, and
outputting the change with time of the index on the basis of the plurality of indexes obtained.

16. A computer-readable medium on which a computer program as defined in any one of Claims 11 to 15 is recorded.
